# EUROPEAN PATENT SPECIFICATION

(11) **EP 1 123 063 B1**
(45) Date of publication and mention of the grant of the patent: **18.03.2015**
(21) Application number: 99950401.2
(22) Date of filing: 29.09.1999
(51) Int. Cl.: A61F 2/07

(54) **EXPANDING INTRALUMINAL DEVICE**
EXPANDIERBARE INTRALUMINALE VORRICHTUNG
DISPOSITIF INTRALUMINAL EXPANSIBLE

(30) Priority: 29.09.1998 AU PP624398
(43) Date of publication of application: 16.08.2001
(73) Proprietor: Gore Enterprise Holdings, Inc., Newark, DE 19714 (US)
(72) Inventor: White, Geoffrey H., East Balmain, NSW 2041 (AU)
(74) Representative: Shanks, Andrew
(86) International application number: PCT/AU1999/000832
(87) International publication number: WO 2000/018322

(56) References cited:
- EP-A1- 0 554 082
- WO-A-96/02211
- WO-A-96/25124
- WO-A-96/39102
- WO-A-97/12562
- WO-A-97/19652
- WO-A1-92/06734
- GB-A- 2 324 729

## Description

### Field of the Invention

The present invention relates to an intraluminal device for use in the treatment of aneurysmal or stenotic disease.

### Background Art

It is known to use intraluminal grafts and stents of various designs for the treatment of aneurysms such as aortic aneurysms, and occlusive diseases affecting the vasculature or other vessels comprising, inter alia, the hepato-biliary and genito-urinary tracts (which are all hereinafter "vessels"). It is known to form such an intraluminal device of a sleeve in which is disposed a plurality of self-expanding wire stents (see Balko A. et al. (1986) Transfemoral Placement of Intraluminal Polyurethane Prosthesis for Abdominal Aortic Aneurysms Journal of Surgical Research 40, 305-309; Mirich D. et al. (1989) Percutaneously Placed Endovascular Grafts for Aortic Aneurysms: Feasibility Study Radiology 170(3), 1033-1037).

In the past, such devices have commonly been used in the treatment of aneurysms. However, it has been recognised that it is within the ambit of some such devices that they also be used to treat stenotic lesions. Whichever the purpose for which an intraluminal device is being used, it has the capacity to be inserted percutaneously through a distal (or proximal) and connecting vessel to that in which the device is to be used. For example, through the femoral artery in a catheter, where the device is intended to be used in the treatment of lesion within the aorta. Upon release of the device from the catheter it may expand to a desirable size, and may extend above and below the lesion thereby bridging the lesion. This method of inserting the device into the body of a patient is applicable where the invention is used in the treatment of aneurysmal disease or stenotic disease.

There are a number of problems associated with such known intraluminal devices. These include the problem of maintaining the device against longitudinal movement along the lumen in which it is placed; and having no specific and/or particular means to engage other instruments or devices, should that be desirable at any stage throughout the life of the device.

Although the first of these problems has been sought to be overcome by prior inventions (see, for example, International Patent Application WO 95/08966; US Patent 5,282,824 entitled Percutaneous Assembly in the name of Gianturco) and also by document WO 97/19652 which discloses an endovascular graft for securement within a vessel, duct or lumen of a living being. The graft includes anchoring projections that are fixed relative to the body of the graft and are arranged for engagement with the wall of the vessel, duct or lumen.

Document WO 96/02211 discloses a mechanically anchoring stent with barbs which remain within the surface of the stent when the stent is in its unexpanded condition but which extend from the surface of the stent when the stent is expanded. The barbs are adapted to engage a graft and/or the inner layers of a blood vessel.

Document WO 97/12562, which represents the closest prior art, describes modular intraluminal prostheses for the treatment of aneurysms. Each prosthetic module comprises one or more interface ends for engaging another module. The interface ends may include barbs which can lock the relative positions of the prosthetic modules at the couplings.

The present inventor has found that there are alternative means to overcome these problems, and has incorporated them into this invention.

Thus the present invention is directed to an alternative form of intraluminal device which, in preferred embodiments, may ameliorate the above problems.

### Summary of the Invention

According a first aspect of the present invention there is provided an intraluminal device according to claim 1.

An intraluminal device is described comprising:
a tubular body with two ends, which body is capable of expanding or being expanded from a radially compressed state to a radially expanded state; and
at least one engagement member which is connected to or integral with a wall of the body at a position located intermediate the ends of the body; wherein,
the connection between the at least one engagement member and the body is such that it will allow the engagement member to occupy a first angular relationship with an adjacent part of the body when the body is radially compressed and a second and different angular relationship with the body when the body is radially expanded.

The present invention consists in an intraluminal device comprising:
a tubular body with two ends, which body is capable of expanding or being expanded from a radially compressed state to a radially expanded state *in vivo*; and,
at least one engagement member which is connected to or integral with a wall of the body; wherein,
the construction and materials of the at least one engagement member and the body are such that the engagement member will occupy a first angular relationship with an adjacent part of the body when the body is radially compressed and a second and different angular relationship with the body when the body is radially expanded.

A method is described for positioning an intraluminal device as described herein, the method comprising the steps of:
introducing a catheter or other delivery device into a vein, artery or other vessel in the body of a patient;
causing the device in a radially compressed state to be carried through the catheter or other delivery device until the intraluminal device extends into the vessel;
causing or allowing the device to expand;
causing or allowing the at least one engagement member to occupy its second angular relationship with the device body; and
withdrawing the catheter or other delivery device along with any other apparatus used to introduce the intraluminal device into the vessel.

The intraluminal device according to this invention may be used in the treatment of aneurysms or stenotic disease. In addition to treating aortic aneurysms the device is particularly suitable for treating aneurysms of the femoral artery, the popliteal artery, the thoracic segment of the aorta, visceral arteries such as the renal and mesenteric arteries, the iliac artery and the sub-clavian artery. Further, in addition to the treatment of stenotic lesions in the peripheral vasculature, the invention may be used in the treatment of, inter alia, vessels comprising the coronary circulation. However the application of the invention for use in the treatment of stenotic disease is not to be understood as limited to the vascular system only, the device may be used to treat stenotic lesions in other vessels including, for example, those comprising the hepato-biliary and genito-urinary tracts.

In cases where the invention is to be used for the treatment of aneurysmal disease, the tubular device body is preferably formed of a thin biocompatible material such as Dacron or PTFE. The tube material is preferably crimped along its length to increase the device's flexibility, however, uncrimped material may be used in suitable circumstances. In preferred embodiments of the invention for use in the treatment of aneurysmal disease, the device body may be formed from a material having a limited amount of diametric elasticity to ensure that it can be expanded into contact with the vessel wall, forming a seal between the wall of the device and the wall of the vessel such that the escape of the vessel contents into the aneurysmal sac is prevented.

In addition, some embodiments for use of the invention in the treatment of aneurysmal disease may be such that the device body includes a stent or a series of spaced apart stents which form a framework to which may be attached an endoluminal graft. The framework may be a plurality of separate, spaced-apart, malleable wires. Each of such wires may have a generally closed sinusoidal or zig-zag shape. The wires are preferably formed of stainless steel or another metal or a plastic which is malleable and is biocompatible. Each wire is preferably woven into the fabric of the device body to integrate the body and the reinforcing wires. This prevents any possibility of the wire reinforcement separating from the device body during introduction of the device and/or throughout its life. If the device body is of a woven material the wires may be interwoven with the device body after its manufacture. If the device body is not woven but is knitted or of an impervious sheet material then the wires may be threaded through suitable holes formed in the device body. Alternatively the stent or stents may be continuous and may be on the radially inner or the radially outer side of the device wall. In either case expansion of the stent or stents will cause the graft to expand and press against the wall of a vessel into which the device has been placed.

In alternative embodiments for use of the invention in the treatment of aneurysmal disease. the wires described above may be held in place by sutures or adhesives or may be sandwiched between layers of a multi-layered tubular device body. In all of the foregoing arrangements the wires are preferably disposed substantially within the device body. It is however, within the ambit of the invention that the wires may be connected to, and be disposed on, the outside surface of the device body.

In cases where the invention is to be used for the treatment of stenotic disease, the tubular device body is preferably formed of a thin biocompatible material such as Nitinol, stainless steel, tantalum or Elgiloy (an alloy of cobalt, chromium and nickel. For the purpose of this application, the device body may be bare or may be coated with a material having an elastic property such that the coating material is capable of covering the device body in both radially compressed and radially expanded states. In preferred embodiments of the invention for use in the treatment of stenotic disease, the device body may also be formed from other suitable biocompatible materials, selected, for best results, on the basis of the material's capacity to withstand the compressive forces of the stenotic lesion and maintain patency of the vessel throughout the life of the device.

In alternative embodiments of the invention for use in the treatment of stenotic disease, the device body may take the form of a cylindrical mesh or may take other forms wherein the wall of the device body is permeable. Permeability of the device wall is not essential. However, if the wall of the device body is permeable, the scope of this invention encompasses all possible patterns chosen for the positioning of the punctures in the wall of the device body, and all possible shapes chosen for each individual perforation of the wall.

In all the foregoing embodiments of the invention, including those for use of the invention in the treatment of aneurysmal disease and those for use of the invention for the treatment of stenotic disease, the length and radially expanded diameter of the device body may be determined by the individual circumstances of the application to which the intraluminal device is to be put. Typically, the vessel will be assessed by X-ray or other similar examination and a suitably dimensioned device selected for that application. Alternatively, the length, radially compressed diameter and radially expanded diameter of the tubular body are predetermined prior to manufacture, in order to provide a device with standard "off-the-shelf" dimensions.

The capacity of the device body to change or to be changed from a radially compressed state to a radially expanded state is an important feature of this invention. It is desirable, for the purpose of introducing the device into the selected vessel, that the device occupies the smallest possible radial diameter along its length. Thus, in a preferred embodiment, the device body will initially be radially compressed and once the invention has been deployed into the selected vessel and positioned appropriately, the device body may be caused to expand, or may be allowed to self-expand.

There are at least three preferred mechanisms whereby the device body may change from a radially compressed state to a radially expanded state. These are: (1) expansion effected by the physical force of an inflating balloon within the device body or by some other mechanically applied force ("mechanical expansion"); (2) self-expansion following the introduction of the device body into the body of a patient, wherein a patient's body temperature causes the temperature of the device body to rise, thereby enabling the device body to self-expand ("thermal expansion"); and, (3) self-expansion following deployment of the invention from the catheter used to introduce the invention into the body of a patient, wherein a property of the material comprising the device body has a "memory" of a preferred shape for the device body in the radially expanded state, such that the device body may "spring" into that state upon release from the catheter ("spring expansion").

In cases where "mechanical expansion" has been selected as the preferred method for causing the device body to change from a radially compressed state to a radially expanded state, a surgeon's intervention will be required to cause that change. Following introduction of a catheter into a selected vessel in the body of a patient, the device may be caused to be carried through the catheter on an inflatable balloon until the device extends into the vessel from the proximal end of the catheter. Once the preferred position for the device is achieved, the balloon may be inflated such that it causes the device body to expand and therefore acquire a radially expanded state. The method of "mechanical expansion" is one in which the surgeon may maintain the rate at which, and extent to which, the device body will expand. It should be noted that in preferred embodiments of the invention, wherein the selected method for changing the device body from a radially compressed state to a radially expanded state is "mechanical expansion", materials such as Dacron or PTFE are particularly amenable for use in the manufacture of the wall of the device body. As an alternative to the use of a balloon to cause mechanical expansion it would be possible to use any one of a number of mechanical arrangements such as a screw jack to bring about expansion of the device body.

Where "thermal expansion" has been selected as the preferred method for causing the device body to change from a radially compressed state to a radially expanded state, such change will not require specific intervention by the surgeon. In this case the device body, upon being introduced into the body of a patient, will undergo an increase in temperature caused by its placement within the body of the patient, and will consequently change its shape such that it acquires a radially expanded state. In embodiments of the invention where "thermal expansion" is the selected method for causing the device body to change from a radially compressed state to a radially expanded state, it may be necessary, before using the invention, to predetermine the desired diameter of the device body in the radially expanded state so that the radial size of the device is appropriate to the circumstances of the particular case. In preferred embodiments of the invention, wherein "thermal expansion" of the device body plays a role, materials such as Nitinol are preferably used in the manufacture of the device body.

In cases where "spring expansion" has been selected as the preferred method for causing the device body to change from a radially compressed state to a radially expanded state, the method for positioning the device is described. In preferred embodiments of the invention where "spring expansion" plays a role, the device body will be manufactured from one or more of a series of alloys which have the capacity to "memorise" their manufactured shape, such that the device, according to this invention, will have a continuous tendency to return to its original shape following any events which cause it to be temporarily deformed. Thus a device, where "spring expansion" is the preferred method for causing the device body to change from a radially compressed state to a radially expanded state, will be manufactured such that it is initially in a radially expanded state. In preferred embodiments of the invention where "spring expansion" is used, the method for positioning such a device will comprise introducing a catheter into a selected vessel; manually compressing the device body into a radially compressed state, and inserting the device with its body maintained in the radially compressed state into the catheter; causing the device to be carried through the catheter until the device extends into the vessel from the proximal end of the catheter (or from some other part of the catheter or other delivery device as may be used to introduce the intraluminal device into the vessel), thereby enabling the device body to "spring" back into a radially expanded state [having been released from the confines of the catheter lumen or any other instrument which had been maintaining the device body in a radially compressed state]; and withdrawing the catheter along with any other apparatus used to introduce the device into the vessel.

The methods described above for causing the expansion of the device body from a radially compressed state to a radially expanded state are by no means representative of an exhaustive list. Many alternative methods, including the use of electromagnetic fields and electric currents may be used.

The presence of engagement members is also an important feature of this invention. The provision of such engagement members will be to act as an attachment, hook or anchor to act as a means for engaging other instruments or devices at any stage throughout the life of the invention and further optionally to prevent the device from moving longitudinally within the vessel following deployment of the invention

In preferred embodiments the invention there will be a plurality of engagement members connected to or integral with the wall of the device body.

In embodiments of the invention wherein the engagement members are connected to the wall of the device body, such connection is preferably created during the manufacture of the invention. The elected method for achieving such connection will primarily depend on the material selected to comprise the engagement members and that selected to comprise the device body. The scope of this invention does include, however, all combinations of selected material for the engagement members and the device body, including the combination in which the material selected for each of these respective components of the invention is the same. Thus, while one means of creating the connection between engagement members and device body may be appropriate for one particular combination of selected materials, an entirely different means may be more appropriate for one of the other possible combinations of selected materials for the respective components of the invention.

In embodiments of the invention wherein the engagement members are integral with a wall of the device body, they will be formed of the same material as that of the device body. In such cases, the construction of the engagement members will depend on the construction of the device body. If for example, in the case of an embodiment of the invention referred to above, the device body has been formed such that it is circumferentially reinforced along its length by a plurality of separate, spaced-apart, malleable wires, each of which has a generally closed sinusoidal or zig-zag shape, the construction of the engagement members may be such that they extend from any one or all of the peaks and/or troughs comprising the sinusoidal or zig-zag shape of those wires.

In all foregoing embodiments with respect to the engagement members of the invention, the engagement members are preferably formed of a material such as Nitinol, stainless steel or one or more of a series of "memory" alloys. However, other materials may also be appropriate for use in the manufacture of the engagement members including plastic materials which may be resorbable. The engagement members may be coated with materials to promote adhesion of cells and/or cell growth to assist in securing the device body in place in the vessel. In addition, although in certain circumstances it may be preferable for all of the engagement members to be either connected to or integral with a wall of the device body, the invention may also be such that only a proportion of the engagement members are connected to a wall of the device body, while the remainder are integral with the same or another wall of the device body. However, the scope of the invention does not limit in any way, the number of possible arrangements by which some of the engagement members are connected to a wall of the device body and others are integral with that or another wall of the device body.

The engagement members may be of differing lengths and may be positioned at different locations on the wall of the device body. In addition to occupying different first and second angular relationships with respective adjacent parts of the device body, each engagement member may also occupy different first and second angular relationships with respect to one another. However, the scope of this invention also includes embodiments wherein the engagement members in either their first or second angular relationships, remain parallel to one another. In one such embodiment of the invention, a group of engagement members may be positioned such that they are spaced apart, surrounding a wall of the device body in the same circumferential plane. The result being that when that group of engagement members change from their first angular relationship to occupying a second angular relationship, together they form a "skirt-shaped" extension of a wall of the device body. Such an arrangement of engagement members may be desirable in certain circumstances.

In preferred embodiments of the invention, the relationship between the device body and the engagement members will be such that when the device body is in a radially compressed state, the respective first angular relationships of the engagement members may be either flat, running along or forming a part of the wall of the device body or, alternatively, the engagement members may project inwardly, within the lumen of the device body. Such first angular relationships of the engagement members is of considerable value in ensuring that the smallest possible diameter along the length of the device body may be maintained when the device body is in a radially compressed state. For reasons already explained it is most desirable, for the purpose of introducing the device into the selected vessel, that the device occupies the smallest radial diameter along its length.

Once the invention has been introduced into the selected vessel and positioned appropriately, the engagement members may be caused to change from occupying their first angular relationship to occupying respective second angular relationships, or the engagement members may make such a change without specific assistance from the surgeon.

There are at least four preferred mechanisms whereby the engagement members may change from having a first angular relationship with an adjacent part of a wall of the device body to having a second angular relationship with an adjacent part of a wall of the device body. These are: (1) change of angular relationship effected by the physical force of an inflating balloon or other mechanical device ("mechanically-aided change"); (2) self-change following the introduction of the invention into the body of a patient, wherein a patient's body temperature causes the temperature of the engagement members to rise, thereby enabling them to change from their first angular relationship to their second angular relationship ("heat-aided change"); (3) self-change following deployment of the invention from the catheter used to introduce the invention into the body of a patient, wherein a property of the material comprising the engagement members has a "memory" of a preferred second angular relationship position, such that the engagement members may "spring" into that position upon release from the catheter ("spring-aided change"); and, (4) change of angular relationship effected by the change in the geometry of the device body as it expands from a radially compressed state to a radially expanded state ("geometry-aided change").

In cases where "mechanically-aided change" has been selected as the preferred method for causing the engagement members to change from their first angular relationship to a second angular relationship, a balloon may be used to cause such change and may therefore be specifically preshaped to suit the particular device with which it will be used. Alternatively however, the balloon to be used may not require any specific manufacturing arrangements which are out of the ordinary. Where the balloon is preshaped, it may be manufactured such that when inflated, it has a series of dimples, between each of which the surface of the balloon does not bulge out as far as it does where the dimples are located. The dimples may be strategically located such that they will push respective engagement members into their second angular relationship when the device is introduced into the body of a patient. Whether or not the balloon is specifically preshaped, the "mechanically-aided change" procedure is effectively the same: once the preferred position for the device is achieved, and the device body has been expanded from a radially compressed state to a radially expanded state as described above, the balloon may be inflated such that its outer surface comes into contact with and presses against the inner surface of the device body and as it continues to inflate, the increasing pressure causes the engagement members to be forced into their respective second angular relationship positions. The method of "mechanically-aided change" is one in which the surgeon may maintain the rate at which, and extent to which, the engagement members will change from a first angular relationship to a second angular relationship. It should be noted that in embodiments of the invention, wherein the selected method for causing the change in angular relationships of the engagement members is "mechanically-aided change", materials such as titanium wire are particularly amenable for use in the manufacture of the invention.

Alternatively the process of mechanically-aided change may be induced by a screw jack or other mechanical means introduced through the catheter or other delivery device along with the intraluminal device.

Where "heat-aided change" is chosen as the preferred method for causing the engagement members to change from a first angular relationship to a second angular relationship, such change will not require specific intervention by the surgeon. In this case, the engagement members, upon the invention being introduced into the body of a patient, will undergo an increase in temperature caused by placement within the body of the patient, and will consequently change their angular relationship such that they acquire a second angular relationship with an adjacent part of the device body as compared to the first. In embodiments of the invention, wherein "heat-aided change" of the angular relationship of the engagement members plays a role, materials such as Nitinol are preferably used in the manufacture of the engagement members. Rather than relying on body heat to induce heat-aided change of the engagement members it would be possible to infuse the device with a heated liquid just prior to, or after, placement of the device in the vessel. It would be possible, for instance, to place the device in a vessel and to actuate the change in the relative position of the engagement members at a later time. Thus, if the device showed signs of moving in the vessel, it may be secured in position by infusing into the vessel a liquid at a temperature above body temperature sufficient to cause the engagement members to change into their second relative positions.

In cases where "spring-aided change" has been selected as the preferred method for causing the angular relationship of the engagement members to change, the method for positioning the device is a defining feature of the invention. In preferred embodiments of the invention where "spring-aided change" plays a role, the engagement members will be manufactured from one or more of a series of alloys which have the capacity to "memorise" their manufactured shape, such that the device, according to this invention, will have a continuous tendency to return to that shape following any events which cause it to be temporarily deformed. Thus a device, where "spring-aided change" is the preferred method for causing the engagement members to change from a first angular relationship to a second angular relationship, will be manufactured such that the engagement members are initially in the second angular relationship position. In preferred embodiments of the invention where "spring-aided change" is used, the method for positioning such a device will comprise introducing a catheter into a selected vessel; manually compressing the engagement members into their first angular relationship positions such that in combination with the device body the invention has the smallest possible radial diameter along its length; inserting the device with the engagement members maintained in their first angular relationship positions into the catheter; causing the device to be carried through the catheter until the device extends into the vessel from the proximal end of the catheter, thereby enabling the engagement members to "spring" back into their respective second angular relationship positions [having been released from the confines of the catheter lumen or any other instrument which had been maintaining them in their first angular relationship positions]; and withdrawing the catheter along with any other apparatus used to introduce the device into the vessel.

In embodiments of the invention wherein the selected method for causing the engagement members to change from a first angular relationship position to a second angular relationship position is "geometry-aided change", the construction of the invention is of particular relevance. In this case, there is a relationship between the expansion of the device body [from a radially compressed state to a radially expanded state] and the change in angular relationship of the engagement members such that the change in the geometry of the device body as it expands causes the engagement members to change from their first angular relationship to a different second angular relationship.

As is the case with the expansion of the device body from a radially compressed state to a radially expanded state, the methods described above for causing the engagement members to change from a first angular relationship to a second angular relationship are by no means representative of an exhaustive list. Many alternative methods, including the use of electromagnetic fields and electric currents may be used.

Having occupied their respective second angular relationships, the engagement members will come into contact with the vessel wall and may penetrate the vessel wall, becoming partly embedded therein, or may perforate the vessel wall. Although the latter of these options is the least desirable, it is unlikely to lead to loss of the contents of the vessel, since the device body [in its radially expanded state] will rest firmly against the vessel wall and act as a 'plug', preventing the escape of the vessel contents through the perforation. The occupation by the engagement members of their respective second angular relationships such that they come in contact with the inside surface of the vessel wall and then being at least partly embedded in the vessel wall will assist in resisting any tendency for the device to move longitudinally within the vessel following deployment.

In embodiments of the invention wherein the occupation by at least one of the engagement members in its respective second angular relationship is such that the engagement member remains within the lumen of the device body, such position of the engagement member will act as a potential means for engaging other instruments or devices, should that be desirable at any stage throughout the life of the invention.

### Brief Description of the Drawings

Hereinafter is an explanation of preferred embodiments of the present invention described with reference to the accompanying diagrams:
Fig. 1 a diagrammatic partially cut-away ventral view of a patient with an aortic aneurysm which has been bridged by an intraluminal device;
Fig. 2 is a longitudinal sectional view of a vessel with a stenotic lesion and a device with its body in a radially compressed state in the lumen of that vessel;
Fig. 3a is a diagrammatic longitudinal view of the device with its body in a radially compressed state and engagement members in their respective first angular relationships;
Fig. 3b is a diagrammatic longitudinal view of the device with its body in a radially expanded state and engagement members in their respective second angular relationships;

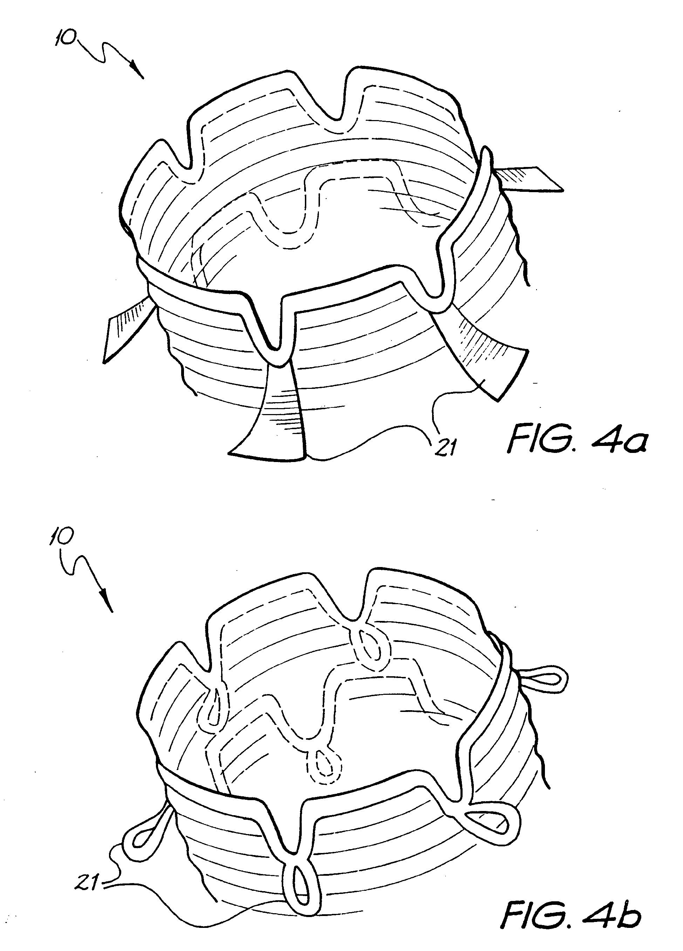

Fig. 4a is a detailed perspective view of a circumferential section of the device in Fig. 3b wherein the engagement members are connected to a wall of the device body;
Fig. 4b is a detailed perspective view of a circumferential section of the device in Fig. 3b wherein the engagement members are integral with a wall of the device body;
Fig. 5 is a longitudinal sectional view of a vessel with two devices according to this invention within the lumen of that vessel. One device is at the distal end of the vessel and has its body in a radially expanded state with some of its engagement members protruding into the wall of the vessel. The other device is at the proximal end of the vessel and has its body in a radially compressed state; and
Fig. 6a is a diagrammatic longitudinal view of the device with its body in a radially compressed state and engagement members integral with the wall of the device body, in their respective first angular relationships;
Fig. 6b is a diagrammatic longitudinal view of the device of Fig. 6a with its body in a radially expanded state and engagement members integral with a wall of the device body, splayed out from the wall of the device body into their respective second angular relationships.
Fig. 7 is a diagrammatic longitudinal view of a variant of the device shown in Figs. 6a and 6b, where each engagement member is surrounded by substantial areas of perforation within the device body.

### Preferred Mode of Carrying the Invention

An intraluminal device is depicted generally as 10 in the accompanying drawings.

In different embodiments, the device 10 may be used in the treatment of aneurysmal disease, such as an aneurysm of the aorta 11 or in the treatment of a stenotic lesion 12 within a vessel 13.

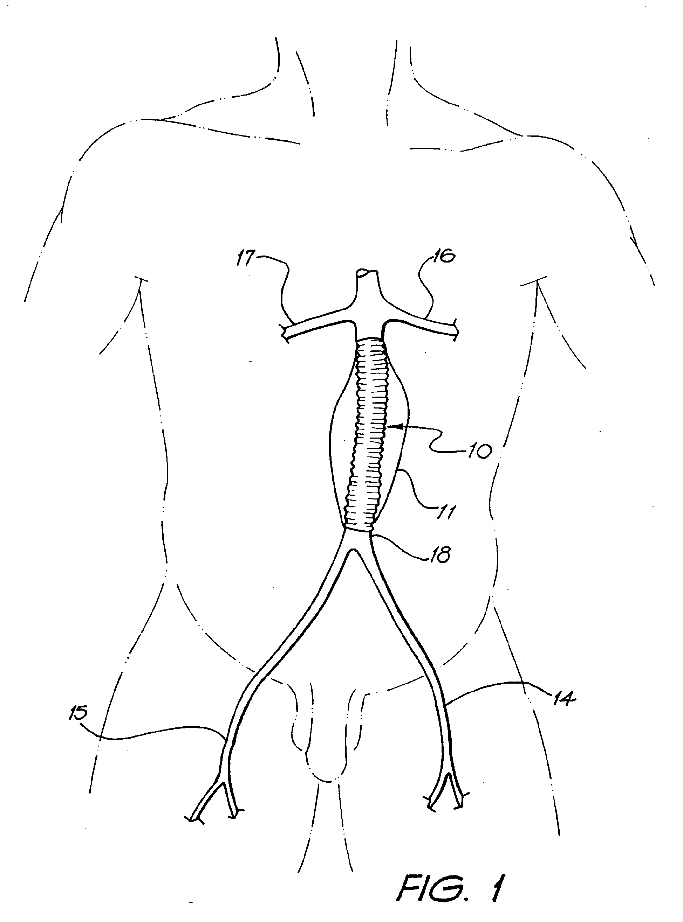

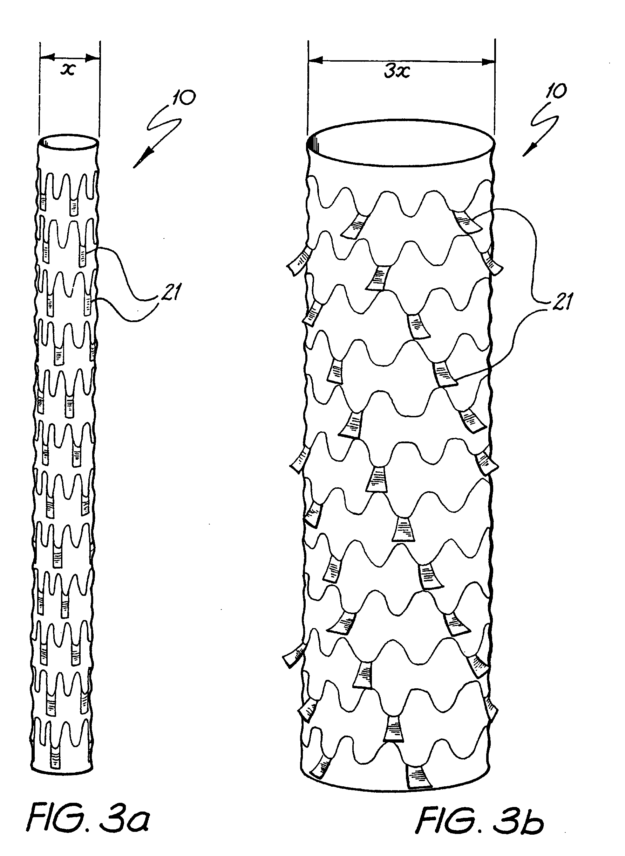

When the device 10 is to be used in the treatment of, for example, an aortic aneurysm, the device 10 is adapted for insertion transfemorally into a patient to achieve bridging and occlusion of the aneurysm present in the aorta 11. As is depicted somewhat simplistically seen in Fig. 1 the aorta 11 is connected to the left and right femoral arteries 14 and 15. The aortic aneurysm is located between the renal arteries 16 and 17 and the bifurcation of the aorta 18. The device 10 is inserted inside a catheter introduced into one of the femoral arteries 14 or 15 in a leg of the patient in a radially compressed state (Fig. 3a). A depiction of the device 10 in a radially compressed state is provided in Fig. 3a. At this instance, the engagement members 21 have a first angular relationship relative to the device body such that they lie flat and run along or form a part of the wall of the device body.

Once the catheter is located appropriately with its proximal end in the aorta 11, the device 10 is deployed from the catheter and is caused or allowed to expand into a radially expanded state (as is depicted in Fig. 3b) so that the wall of the device comes into intimate contact with the luminal wall of the aorta 11. As the body of the device 10 expands from a radially compressed state (Fig. 3a) to a radially expanded state (Fig. 3b) the engagement members 21 may be caused or allowed to change from their first angular relationship (Fig. 3a) to a different second angular relationship (Figs. 3b). In their respective second angular relationships, the engagement members 21 may come into contact with the vessel wall 19 and may penetrate the vessel wall 19, becoming partly embedded therein, or may perforate the vessel wall 19. The device then bridges the aneurysm, isolating any thrombosis or gelatinous material associated with the aneurysm outside the device 10 and reducing the risk of embolisation. The device is prevented from longitudinal movement within the aorta by virtue of the connection between the engagement members 21 and the vessel wall 19.

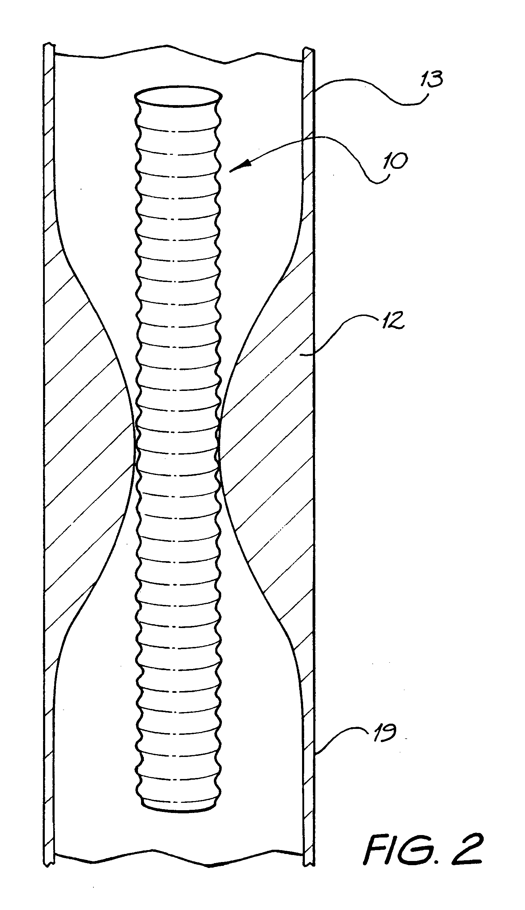

When the device 10 is to be used in the treatment of a stenotic lesion, the device 10 is adapted to be inserted into the selected vessel 13 in a patient to achieve radial expansion of the stenosis and patency of the vessel 13. As is seen in Fig. 2, the device is inserted in its radially compressed state (Fig. 3a). It is desirable, for the purpose of introducing the device 10 into the selected vessel 13, for it to occupy the smallest possible diameter along its length. In this case, the device 10 has the capacity to be introduced percutaneously through a distal (or proximal) and connecting vessel to that in which the device is to be deployed. As above, a catheter may be used to introduce the device 10 into the patient.

Once the device 10 is positioned appropriately, it may be caused or allowed to expand to a radially expanded state (Fig. 3b), and in so doing it will cause the stenotic lesion 12 itself to radially expand. The device 10 will then maintain that position for the remainder of its life, thereby ensuring patency of the vessel. The engagement members 21 will also change their position from a first angular relationship to a different second angular relationship and perform their function as described above.

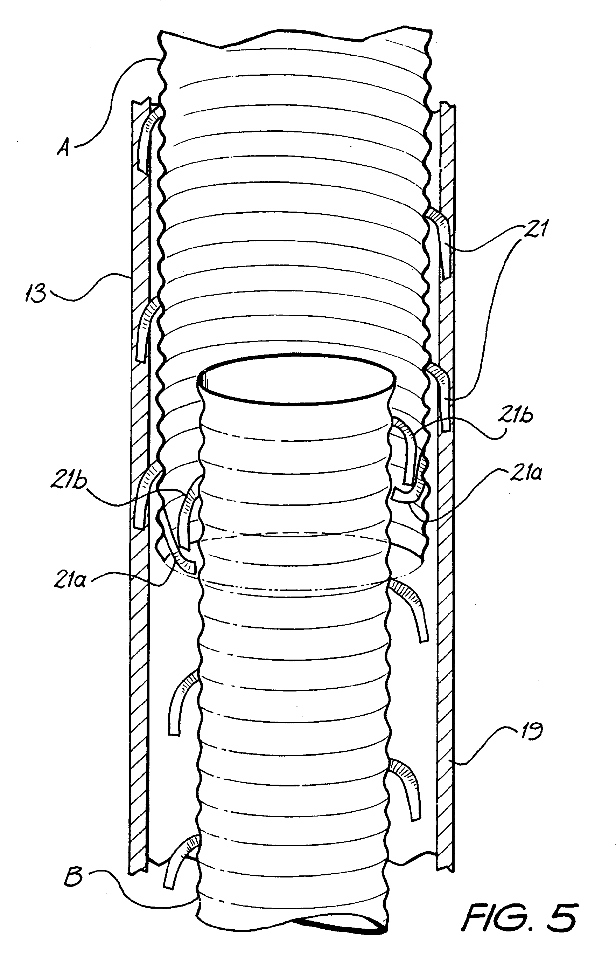

Whether the device 10 is being used in the treatment of aneurysmal disease or in the treatment of a stenotic lesion, it may be appropriate for the second angular relationship adopted by at least one of the engagement members 21 to be such that the engagement member remains within the lumen of the device 10. Such position of the engagement member will act as a potential means for engaging other instruments or devices passed through the lumen of the device 10, should that be desirable at any stage throughout the life of the invention. As can be seen in Fig. 5, two engagement members 21a at the proximal end of a first device A project inwardly within the lumen of the device 10. Device B may be introduced into the vessel for the purpose of interconnecting with and therefore lengthening or reinforcing device A. In Fig. 5, device B is depicted in a radially compressed state (such as is depicted in Fig. 3a). As illustrated, the respective spatial positions of two engagement members 21b at the distal end of device B correspond with the respective spatial positions of the two engagement members 21a at the proximal end of device A, such that the said engagement members 21a and 21b of the two devices A and B may ultimately engage with one another. As device B expands to a radially expanded state, its engagement members 21b will acquire the second angular relationships, thereby interlocking with the engagement members 21a of device A and providing an effective means to keep the two devices A and B interconnected.

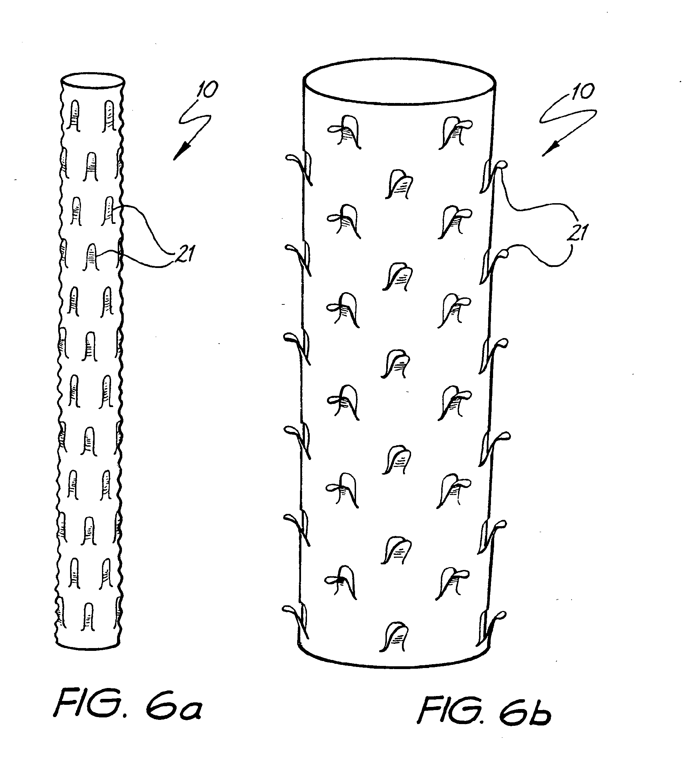

Figs. 6a and 6b illustrate a further embodiment of the device 10 in its radially compressed and radially expanded states respectively. In this embodiment, the body of the device 10 is preferably formed of Nitinol; and the engagement members 21 are continuous with the wall of the device 10, but for a small incision in the wall of the device defining each of the individual engagement members' 21 shapes. Further, "thermal expansion" is the preferred method for causing the device body 10 to change from a radially compressed state (Fig. 6a) to a radially expanded state (Fig. 6b); and similarly "heat-aided change" is the preferred method for causing the engagement members 21 to change from a first angular relationship to a different second angular relationship. This embodiment of the device 10 may be manufactured so that the heat pre-treatment of the Nitinol is such that the engagement members 21 have the capacity, following a rise in temperature, to splay out from a wall of the device. Thus, although the radially expanded state (Fig. 6b) of the device 10 may be of a fixed diameter, the particular make-up of the components of the wall comprising the engagement members 21, is such that the engagement members 21 may extend further out from a wall of the device body.

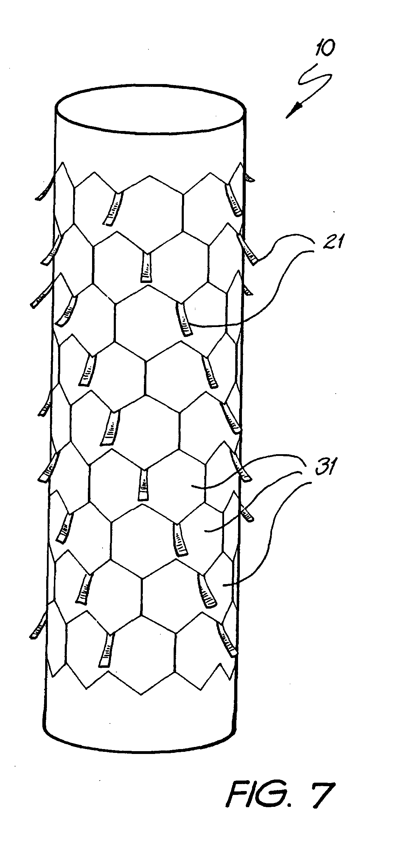

Fig. 7 depicts yet another embodiment of a device 10 wherein there is no specific material covering or lining for the device 10. In this embodiment, the areas between the wires forming the framework of the device body, and hence the areas surrounding the origins of each of the engagement members 21, essentially act as perforations 31, through which, for example, vessel 13 contents may freely pass. A device 10 according to this embodiment is most preferably used in the treatment of stenotic disease rather than in the treatment of aneurysmal disease.

It will be appreciated by persons skilled in the art that numerous variations and/or modifications may be made to the invention as shown in the specific embodiments without departing from the scope of the invention as claimed. The present embodiments are, therefore, to be considered in all respects as illustrative and not restrictive.

## Claims

1. An intraluminal device (10) comprising:
a tubular body (A) with two ends, which body is capable of expanding or being expanded from a radially compressed state to a radially expanded state *in vivo;* and,
at least one engagement member (21a) which is connected to or integral with a wall of the body,
the construction and materials of the at least one engagement member (21a) and the body are such that the engagement member (21a) will occupy a first angular relationship with an adjacent part of the body when the body is radially compressed and a second and different angular relationship with the body when the body is radially expanded,
further comprising a second tubular body (B) having at least one second engagement member (21 b),
wherein the at least one engagement member (21 a) is configured to engage the second engagement member (21 b) to maintain the tubular body (A) and the second tubular body (B) in an interconnected configuration.

2. The intraluminal device (10) according to claim 1, wherein the tubular body is formed of a plurality of separate, spaced apart, malleable wires to which may be attached an endoluminal graft, and optionally wherein the spaced apart wires have a generally closed sinusoidal or zig-zag shape and the engagement members extend from any one or all of the peaks and/or troughs comprising the sinusoidal or zig-zag shape of those wires.

3. The intraluminal device (10) according to claim 2, wherein the wires are continuous around the circumference of the device.

4. The intraluminal device (10) according to claim 2, wherein the wires are substantially on a radially inner surface of the device.

5. The intraluminal device (10) according to any one of the preceding claims, wherein the body is formed of at least two layers of material.

6. The intraluminal device (10) according to claim 5, wherein the wires are sandwiched between said two layers.

7. The intraluminal device (10) according to claim 2, wherein the wires are formed of a thin biocompatible material selected from the group comprising Nitinol, stainless steel, Tantalum or an alloy of cobalt, chromium and nickel.

8. The intraluminal device (10) according to any one of the preceding claims, wherein an outer surface of the device body is additionally coated with a material having an elastic property, such that the coating material is capable of covering the device body in both radially compressed and radially expanded states.

9. The intraluminal device (10) according to any one of the preceding claims, wherein a wall of the body is permeable.

10. The intraluminal device (10) according to claim 9, wherein the permeability of the wall of the body is created by a plurality of perforations (31) formed in any pattern in the wall of the body.

11. The intraluminal device (10) according to any one of the preceding claims, wherein the engagement members (21) are coated with materials which promote adhesion cells and/or cell growth.

12. The intraluminal device (10) according to any one of the preceding claims, wherein the engagement members (21) are all of the same length.

13. The intraluminal device (10) according to any one of the preceding claims, wherein each engagement member (21 a) is constructed to allow a different first and second angular relationship (21 b) to the body compared to that of at least one of the other engagement members (21).

14. The intraluminal device (10) according to any one of the preceding claims, wherein the relationship between the device body (10) and the engagement members (21) is such that when the device body (10) is in a radially compressed state, at least one of the engagement members (21) projects inwardly, within the lumen of the device body (10).

15. The intraluminal device (10) according to any one of the preceding claims, wherein the engagement members (21) are caused to change from their first respective angular relationship to their second respective angular relationship by mechanically-aided change.

16. The intraluminal device (10) according to claim 15, wherein the mechanically-aided change is effected by means of an inflatable balloon.

17. The intraluminal device (10) according to one of claims 1 to 14, wherein the engagement members (21) are allowed to change from their respective angular relationships to their second respective angular relationships by heat-aided change.

18. The intraluminal device (10) according to claim 17, wherein the introduction of the device (10) into a vessel in the body of a patient is adequate to change its temperature sufficiently to cause the engagement members to change from their first respective angular relationships to their second respective angular relationships.

19. The intraluminal device (10) according to one of claims 1 to 14, wherein the engagement members (21) are allowed to change from their first respective angular relationships to their second respective angular relationships by memory-aided change.

20. The intraluminal device (10) according to claim 19, wherein the device (10) is manufactured such that the engagement members (21) are initially in their second respective angular relationships.

21. The intraluminal device (10) according to claim 20, wherein manual compression is adequate to cause the engagement members (21) to change from their second respective angular relationship, said manual compression being only adequate to cause the engagement members to undergo said change only temporarily, such that upon release of said manual compression, the engagement members immediately return to their respective second angular relationships.

22. The intraluminal device (10) according to any one of claims 1 to 14, wherein the engagement members (21) are allowed to change from their first respective angular relationships to their second respective angular relationships by geometry-aided change.

23. The intraluminal device (10) according to any of the preceding claims, wherein the device (10) is in a radially compressed state during its introduction into a vessel.

24. The intraluminal device (10) according to any one of the preceding claims wherein the tubular body (10) is caused to change from a radially compressed state to a radially expanded state by mechanical expansion.

25. The intraluminal device (10) according to claim 24, wherein mechanical expansion is effected by means of an inflatable balloon.

## Patentansprüche

1. Intraluminale Vorrichtung (10), die aufweist:
einen rohrförmigen Körper (A) mit zwei Enden, wobei der Körper expandieren kann oder aus einem radial zusammengedrückten Zustand in einen radial expandierten Zustand in vivo expandiert wird; und
mindestens ein Eingriffselement (21a), das mit einer Wand des Körpers verbunden oder zusammenhängend damit ist,
wobei die Konstruktion und die Materialien des mindestens einen Eingriffselementes (21a) und des Körpers so sind, dass das Eingriffselement (21a) eine erste Winkelbeziehung mit einem benachbarten Teil des Körpers, wenn der Körper radial zusammengedrückt wird, und eine zweite und abweichende Winkelbeziehung mit dem Körper einnehmen wird, wenn der Körper radial expandiert wird,
die außerdem einen zweiten rohrförmigen Körper (B) mit mindestens einem zweiten Eingriffselement (21b) aufweist,
wobei das mindestens eine Eingriffselement (21a) ausgebildet ist, um mit dem zweiten Eingriffselement (21b) in Eingriff zu kommen, um den rohrförmigen Körper (A) und den zweiten rohrförmigen Körper (B) in einer miteinander verbundenen Konfiguration zu halten.

2. Intraluminale Vorrichtung (10) nach Anspruch 1, bei der der rohrförmige Körper aus einer Vielzahl von separaten, beabstandeten, verformbaren Drähten gebildet wird, an denen ein endoluminales Transplantat befestigt werden kann, und wobei wahlweise die beabstandeten Drähte eine im Allgemeinen geschlossene sinusförmige oder Zickzackform aufweisen, und wobei sich die Eingriffselemente von einem oder allen Scheitelpunkten und/oder Tiefpunkten erstrecken, die die sinusförmige oder Zickzackform jener Drähte aufweisen.

3. Intraluminale Vorrichtung (10) nach Anspruch 2, bei der die Drähte kontinuierlich um den Umfang der Vorrichtung verlaufen.

4. Intraluminale Vorrichtung (10) nach Anspruch 2, bei der sich die Drähte im Wesentlichen auf einer radial inneren Fläche der Vorrichtung befinden.

5. Intraluminale Vorrichtung (10) nach einem der vorhergehenden Ansprüche, bei der der Körper aus mindestens zwei Materialschichten gebildet wird.

6. Intraluminale Vorrichtung (10) nach Anspruch 5, bei der die Drähte schichtartig zwischen den zwei Schichten angeordnet sind.

7. Intraluminale Vorrichtung (10) nach Anspruch 2, bei der die Drähte aus einem dünnen, biokompatiblen Material gebildet werden, ausgewählt aus der Gruppe, die Nitinol, nichtrostenden Stahl, Tantal oder eine Legierung von Kobalt, Chrom und Nickel aufweist.

8. Intraluminale Vorrichtung (10) nach einem der vorhergehenden Ansprüche, bei der eine äußere Fläche des Körpers der Vorrichtung zusätzlich mit einem Material mit einer elastischen Eigenschaft beschichtet ist, so dass das Beschichtungsmaterial den Körper der Vorrichtung in sowohl dem radial zusammengedrückten als auch dem radial expandierten Zustand bedecken kann.

9. Intraluminale Vorrichtung (10) nach einem der vorhergehenden Ansprüche, bei der eine Wand des Körpers durchlässig ist.

10. Intraluminale Vorrichtung (10) nach Anspruch 9, bei der die Durchlässigkeit der Wand des Körpers durch eine Vielzahl von Perforationen (31) erzeugt wird, die in einem Muster in der Wand des Körpers ausgebildet sind.

11. Intraluminale Vorrichtung (10) nach einem der vorhergehenden Ansprüche, bei der die Eingriffselemente (21) mit Materialien beschichtet sind, die die Adhäsionszellen und/oder das Zellenwachstum begünstigen.

12. Intraluminale Vorrichtung (10) nach einem der vorhergehenden Ansprüche, bei der die Eingriffselemente (21) alle von der gleichen Länge sind.

13. Intraluminale Vorrichtung (10) nach einem der vorhergehenden Ansprüche, bei der ein jedes Eingriffselement (21a) konstruiert ist, um eine unterschiedliche erste und zweite Winkelbeziehung (21b) zum Körper zu gestatten, verglichen mit der von mindestens einem der anderen Eingriffselemente (21).

14. Intraluminale Vorrichtung (10) nach einem der vorhergehenden Ansprüche, bei der die Beziehung zwischen dem Körper der Vorrichtung (10) und den Eingriffselementen (21) so ist, dass, wenn sich der Körper der Vorrichtung (10) in einem radial zusammengedrückten Zustand befindet, mindestens eines der Eingriffselemente (21) nach innen innerhalb des Hohlraumes des Körpers der Vorrichtung (10) vorsteht.

15. Intraluminale Vorrichtung (10) nach einem der vorhergehenden Ansprüche, bei der die Eingriffselemente (21) veranlasst werden, sich von ihrer ersten jeweiligen Winkelbeziehung zu ihrer zweiten jeweiligen Winkelbeziehung mittels einer mechanisch unterstützen Veränderung zu verändern.

16. Intraluminale Vorrichtung (10) nach Anspruch 15, bei der die mechanisch unterstützte Veränderung mittels eines aufblasbaren Ballons bewirkt wird.

17. Intraluminale Vorrichtung (10) nach einem der Ansprüche 1 bis 14, bei der sich die Eingriffselemente (21) von ihren jeweiligen Winkelbeziehungen in ihre zweiten jeweiligen Winkelbeziehungen mittels einer wärmeunterstützten Veränderung verändern können.

18. Intraluminale Vorrichtung (10) nach Anspruch 17, bei der die Einführung der Vorrichtung (10) in ein Gefäß im Körper eines Patienten angemessen ist, um ihre Temperatur ausreichend zu verändern, um zu bewirken, dass sich die Eingriffselemente von ihren ersten jeweiligen Winkelbeziehungen in ihre zweiten jeweiligen Winkelbeziehungen verändern.

19. Intraluminale Vorrichtung (10) nach einem der Ansprüche 1 bis 14, bei der sich die Eingriffselemente (21) von ihren ersten jeweiligen Winkelbeziehungen in ihre zweiten jeweiligen Winkelbeziehungen mittels einer speicherunterstützten Veränderung verändern können.

20. Intraluminale Vorrichtung (10) nach Anspruch 19, bei der die Vorrichtung (10) so hergestellt wird, dass sich die Eingriffselemente (21) anfangs in ihren zweiten jeweiligen Winkelbeziehungen befinden.

21. Intraluminale Vorrichtung (10) nach Anspruch 20, bei der ein manuelles Zusammendrücken angemessen ist, um zu bewirken, dass sich die Eingriffselemente (21) aus ihrer zweiten jeweiligen Winkelbeziehung verändern, wobei das manuelle Zusammendrücken nur angemessen ist, um zu bewirken, dass sich die Eingriffselemente der Veränderung nur zeitweilig unterziehen, so dass die Eingriffselemente bei Freigabe des manuellen Zusammendrückens sofort in ihre jeweiligen zweiten Winkelbeziehungen zurückkehren.

22. Intraluminale Vorrichtung (10) nach einem der Ansprüche 1 bis 14, bei der sich die Eingriffselemente (21) aus ihren ersten jeweiligen Winkelbeziehungen in ihre zweiten jeweiligen Winkelbeziehungen mittels einer geometrieunterstützten Veränderung verändern können.

23. Intraluminale Vorrichtung (10) nach einem der vorhergehenden Ansprüche, wobei sich die Vorrichtung (10) in einem radial zusammengedrückten Zustand während ihrer Einführung in ein Gefäß befindet.

24. Intraluminale Vorrichtung (10) nach einem der vorhergehenden Ansprüche, bei der bewirkt wird, dass sich der rohrförmige Körper (10) von einem radial zusammengedrückten Zustand in einen radial expandierten Zustand mittels einer mechanischen Expansion verändert.

25. Intraluminale Vorrichtung (10) nach Anspruch 24, bei der die mechanische Expansion mittels eines aufblasbaren Ballons bewirkt wird.

## Revendications

1. Dispositif intraluminal (10), comprenant :
un corps tubulaire (A) avec deux extrémité, ce corps étant capable de se dilater ou étant dilaté d'un état à compression radiale vers un état à dilatation radiale in vivo ; et
au moins un moyen d'engagement (21a), connecté à une paroi du corps ou faisant partie intégrante de celle-ci ;
la construction et les matériaux du au moins un élément d'engagement (21a) et du corps étant tels que l'élément d'engagement (21a) occupe une première relation angulaire par rapport à une partie adjacente du corps lorsque le corps est comprimé radialement, et une deuxième relation angulaire différente par rapport au corps lorsque le corps est dilaté radialement ;
comprenant en outre un deuxième corps tubulaire (B), comportant au moins un deuxième élément d'engagement (21b) ;
dans lequel le au moins un élément d'engagement (21a) est destiné à s'engager dans le deuxième élément d'engagement (21b) pour maintenir le corps tubulaire (A) et le deuxième corps tubulaire (B) dans une configuration interconnectée.

2. Dispositif intraluminal (10) selon la revendication 1, dans lequel le corps tubulaire est formé à partir de plusieurs fils séparés, espacés et malléables, sur lesquels peut être fixée une greffe endoluminale, et dans lequel les fils espacés ont optionnellement une forme sinusoïdale fermée ou en zigzag, les éléments d'engagement s'étendant à partir d'un quelconque ou de tous les pics et/ou les creux comprenant la forme sinusoïdale ou en zigzag de ces fils.

3. Dispositif intraluminal (10) selon la revendication 2, dans lequel les fils s'étendent en continu autour de la circonférence du dispositif.

4. Dispositif intraluminal (10) selon la revendication 2, dans lequel les fils sont agencés pour l'essentiel sur une surface radialement interne du dispositif.

5. Dispositif intraluminal (10) selon l'une quelconque des revendications précédentes, dans lequel le corps est formé à partir d'au moins deux couches de matériau.

6. Dispositif intraluminal (10) selon la revendication 5, dans lequel les fils sont agencés en sandwich entre lesdites deux couches.

7. Dispositif intraluminal (10) selon la revendication 2, dans lequel les fils sont formés à partir d'un fin matériau biocompatible sélectionné dans le groupe constitué de Nitinol, d'acier inoxydable, de tantale ou d'un quelconque alliage de cobalt, de chrome et de nickel.

8. Dispositif intraluminal (10) selon l'une quelconque des revendications précédentes, dans lequel une surface externe du corps du dispositif est en plus revêtue d'un matériau présentant une propriété élastique, de sorte que le matériau de revêtement est capable de recouvrir le corps du dispositif dans les deux états, l'état à compression radiale et l'état à dilatation radiale.

9. Dispositif intraluminal (10) selon l'une quelconque des revendications précédentes, dans lequel une paroi du corps est perméable.

10. Dispositif intraluminal (10) selon la revendication 9, dans lequel la perméabilité de la paroi du corps est établie par plusieurs perforations (31) formées dans un quelconque motif dans la paroi du corps.

11. Dispositif intraluminal (10) selon l'une quelconque des revendications précédentes, dans lequel les éléments d'engagement (21) sont revêtus de matériaux favorisant l'adhésion des cellules et/ou la croissance des cellules.

12. Dispositif intraluminal (10) selon l'une quelconque des revendications précédentes, dans lequel les éléments d'engagement (21) ont tous la même longueur.

13. Dispositif intraluminal (10) selon l'une quelconque des revendications précédentes, dans lequel chaque élément d'engagement (21a) est construit de sorte à permettre l'établissement de première et deuxième relations angulaires (21b) par rapport au corps, différentes d'au moins une relation des autres éléments d'engagement (21).

14. Dispositif intraluminal (10) selon l'une quelconque des revendications précédentes, dans lequel la relation entre le corps du dispositif (10) et les éléments d'engagement (21) est telle que lorsque le corps du dispositif (10) se trouve dans un état radialement comprimé, au moins un des éléments d'engagement (21) déborde vers l'intérieur, dans la lumière du corps du dispositif (10).

15. Dispositif intraluminal (10) selon l'une quelconque des revendications précédentes, dans lequel les éléments d'engagement (21) sont entraînés à passer de leur première relation angulaire respective vers leur deuxième relation angulaire respective par un changement à assistance mécanique.

16. Dispositif intraluminal (10) selon la revendication 15, dans lequel le changement à assistance mécanique est effectué par l'intermédiaire d'un ballonnet gonflable.

17. Dispositif intraluminal (10) selon l'une des revendications 1 à 14, dans lequel les éléments d'engagement (21) sont autorisés à passer de leurs premières relations angulaires respectives vers leurs deuxièmes relations angulaires respectives par un changement à assistance thermique.

18. Dispositif intraluminal (10) selon la revendication 17, dans lequel l'introduction du dispositif (10) dans un vaisseau dans le corps d'un patient est adaptée pour entraîner un changement suffisant de la température pour entraîner le passage des éléments d'engagement de leurs premières relations angulaires respectives vers leurs deuxièmes relations angulaires respectives.

19. Dispositif intraluminal (10) selon l'une des revendications 1 à 14, dans lequel les éléments d'engagement (21) sont autorisés à passer de leurs premières relations angulaires respectives vers leurs deuxièmes relations angulaires respectives par un changement assisté par mémoire.

20. Dispositif intraluminal (10) selon la revendication 19, dans lequel le dispositif (10) est fabriqué de sorte que les éléments d'engagement (21) se trouvent initialement dans leurs deuxièmes relations angulaires respectives.

21. Dispositif intraluminal (10) selon la revendication 20, dans lequel une compression manuelle est adaptée pour entraîner le changement des éléments d'engagement (21) à partir de leur deuxième relation angulaire respective, ladite compression manuelle étant uniquement adaptée pour entraîner les éléments d'engagement à subir ledit changement uniquement de manière temporaire, de sorte que lors du dégagement de ladite compression manuelle, les éléments d'engagement reviennent immédiatement vers leurs deuxièmes relations angulaires respectives.

22. Dispositif intraluminal (10) selon l'une quelconque des revendications 1 à 14, dans lequel les éléments d'engagement (21) sont autorisés à passer de leurs premières relations angulaires respectives vers leurs deuxièmes relations angulaires respectives par un changement à assistance géométrique.

23. Dispositif intraluminal (10) selon l'une quelconque des revendications précédentes, dans lequel le dispositif (10) se trouve dans un état radialement comprimé lors de son introduction dans un vaisseau.

24. Dispositif intraluminal (10) selon l'une quelconque des revendications précédentes, dans lequel le corps tubulaire (10) est entraîné à passer d'un état radialement comprimé vers un état radialement dilaté par dilatation mécanique.

25. Dispositif intraluminal (10) selon la revendication 24, dans lequel la dilatation mécanique est effectuée par l'intermédiaire d'un ballonnet gonflable.
